# EUROPÄISCHE PATENTSCHRIFT

(11) **EP 3 432 971 B1**
(45) Veröffentlichungstag und Bekanntmachung des Hinweises auf die Patenterteilung: **26.02.2020**
(21) Anmeldenummer: 17712111.8
(22) Anmeldetag: 20.03.2017
(51) Int. Cl.: A61M 39/24, A61M 5/28, B01L 3/00, A61B 5/15, A61B 5/153, A61B 5/154, A61B 10/00

(54) **VORRICHTUNG ZUR ENTNAHME VON KÖRPERFLÜSSIGKEITEN, INSBESONDERE BLUT**
DEVICE FOR DRAWING BODILY FLUIDS, IN PARTICULAR BLOOD
DISPOSITIF POUR LE PRÉLÈVEMENT DE LIQUIDES CORPORELS, EN PARTICULIER DE SANG

(30) Priorität: 22.03.2016 DE 102016204702
(43) Veröffentlichungstag der Anmeldung: 30.01.2019
(73) Patentinhaber: Sarstedt AG & Co. KG, 51588 Nümbrecht (DE)
(72) Erfinder: SCHUSTER, Rainer, Wipperfürth 51688 (DE)
(74) Vertreter: Grosse, Wolf-Dietrich Rüdiger
(86) Internationale Anmeldenummer: PCT/EP2017/056529
(87) Internationale Veröffentlichungsnummer: WO 2017/162574

(56) Entgegenhaltungen:
- EP-A1- 2 649 937
- EP-A2- 0 841 094
- WO-A1-92/20449
- JP-A- 2005 287 955
- US-A- 4 112 924
- US-A- 4 134 512
- US-A- 4 256 120

## Beschreibung

Die Erfindung betrifft eine Vorrichtung zur Ent- und / oder Aufnahme von Körperflüssigkeiten, insbesondere Blut, umfassend ein Röhrchen, das an seinem oberen, offenen Ende mit einer Kappe dicht verschlossen ist, die mit einer Rückstromsperre ausgebildet ist und zum Halten einer von einem Nadelhalter getragenen Kanüle bzw. Doppelkanüle einen domartigen Ansatz aufweist, der an seinem oberen Ende in einer Längsbohrung eine von der Kanüle durchstechbare Membrane aufnimmt.

Die Druckschrift EP 2 649 937 A1 offenbart eine Vorrichtung zur Ent- und/ oder Aufnahme von Körperflüssigkeiten umfassend ein Röhrchen, das an seinem oberen, offenen Ende mit einer Kappe vakuumdicht verschlossen ist. Die Kappe weist einen domartigen Ansatz zum Halten einer von einem Nadelhalter getragenen Kanüle bzw. Doppelkanüle auf. Die Kappe ist an ihrem vorderen Ende von einer von der Kanüle durchstechbaren Membrane verschlossen.

Um ein Zurückströmen einer Blutprobe während der Entnahme und / oder ein Austreten von Blut nach Abschluss der Entnahme zu verhindern, ist es aus der DE 35 41 335 A1 bekannt, in dem auf das Röhrchen schraub- oder steckbaren Teil der Verschlusskappe einer Blutentnahmevorrichtung ein Rückschlagventil vorzusehen. Dieses besteht aus einer elastisch verformbaren Membrane mit einer Ventilöffnung und einem innen an der Verschlusskappe angeformten Ventilsitz. Der domartige Ansatz ist als Konus mit einer in das Probenröhrchen mündenden Längsbohrung ausgebildet, der sich die Rückstromsperre in dem Schraub- bzw. Steckteil der Kappe anschließt. Bei einem niemals völlig auszuschließenden Störverhalten der Rückstromsperre kann sich die gesamte Längsbohrung des domartigen Ansatzes mit unerwünschter Flüssigkeit füllen.

Die Rückstromsperre besteht aus einer elastisch verformbaren Membrane mit einer Ventilöffnung und einem an die Verschlusskappe angeformten, kreisrunden Ventilsitz. Im Steckteil bzw. Stopfen der Verschlusskappe ist ein die Membrane stützender Ring vorgesehen, wobei die Membrane und der Stützring einteilig ausgebildet sein können. Die Dichtfläche der Membrane in ihrem Ventilsitz im Stopfen ist groß und entspricht abgesehen von der Wanddicke des Stützrings nahezu dem Durchmesser der den Stützring mit der Membrane aufnehmenden Innenbohrung des Stopfens der Verschlusskappe. Damit sich die Membrane gegen den Ventilsitz legt und ein Zurückströmen verhindert, ist daher ein hoher Druck erforderlich.

Eine eine besonders große Funktionssicherheit gewährleistende Rückstromsperre wird dann erforderlich, wenn das Blutentnahmeröhrchen, das kann ein Vakuumröhrchen oder ein nach dem Aspirationsprinzip durch Ziehen einer Kolbenstange wirkendes Röhrchen sein, mit für einen Patienten schädlichen Präparatslösungen vorbereitet ist, mit deren gefährlichen Substanzen der Patient oder das Bedienpersonal während der Blutentnahme, aber auch vorher und nachher, ungewollt nicht in Berührung kommen darf.

Das gilt so gleichermaßen bei der Entnahme aus einem Blutspendebeutel oder bei der Probennahme aus einem Sammelbehälter für beispielsweise Urin.

Der Erfindung liegt die Aufgabe zugrunde, bei einer Vorrichtung der eingangs genannten Art die Wirksamkeit der Rückstromsperre und die Betriebssicherheit bei der Flüssigkeitsentnahme zu verbessern.

Diese Aufgabe wird erfindungsgemäß dadurch gelöst, dass die Rückstromsperre in dem domartigen Ansatz mit einem in der Längsbohrung unterhalb der durchstechbaren Membrane vorgesehenen Haltemittel angeordnet ist, wobei das Haltemittel mit mindestens einer durch die Rückstromsperre nach der Flüssigkeitsentnahme zum Röhrcheninneren verschlossenen Durchflussöffnung ausgebildet ist.

Hierdurch wird nicht nur erreicht, dass allenfalls eine auf das geringstmögliche Minimum begrenzte Flüssigkeitsmenge zurückströmen kann, sondern es wird gleichzeitig der zum Schließen der Rückstromsperre erforderliche Druck deutlich herabgesetzt und damit die Funktionssicherheit entsprechend erhöht. Die Durchflussöffnungen des Haltemittels gewährleisten dabei im Zusammenspiel mit der Längsbohrung des domartigen Ansatzes das Einströmen der entnommenen Flüssigkeit in das Probenröhrchen.

Die abhängigen Ansprüche geben vorteilhafte Ausgestaltungen bzw. Varianten sowohl der Rückstromsperre als auch des Haltemittels an.

Die Rückstromsperre kann eine durch eine Federkraft in Schließposition beaufschlagte, in einem aus zwei aufeinanderfolgenden, durch Klemmung und / oder Verrastung in der Längsbohrung des domartigen Ansatz festgelegten Hülsen bestehenden Haltemittel angeordnete Kugel sein. Die untere Hülse ist mit einem kronenartigen Kopfrand ausgebildet, in den die Kugel bei der Flüssigkeitsentnahme gegen die Federkraft gedrückt wird. Die damit freie Durchflussöffnung der oberen Hülse steht dann in Verbindung mit den von der unteren Hülse durch die zwischen den Kronenstegen vorhandenen Lücken bereitgestellten Durchflussöffnungen.

Bei einer sogenannten Duckbrill-Ausführung der Rückstromsperre läßt sich zwischen zwei Hülsen ein mit einem schnabelförmigen Ventilkopf in die untere Hülse hineinragendes Ringelement anordnen. Der Ventilkopf des zwischen den beiden Hülsen geklemmten Ringelementes weitet sich bei der Flüssigkeitsentnahme und anschließend schließt sich der Schnabel bzw. Ventilkopf wieder.

Eine andere Ausführung sieht als Haltemittel eine Einzelhülse vor, die endseitig, unterhalb eines mit außermittigen Durchflussöffnungen versehenen Bodens einen Ringraum aufweist, der als Rückstromsperre eine von mittig am Boden angeordneten Umbördelungsmittel geklemmte, in dem Ringraum die außermittigen Durchflussöffnungen überlappende, elastische Membranscheibe aufnimmt, wobei die Membranscheibe während der Flüssigkeitsentnahme nach unten wegklappt und die Durchflussöffnungen freigibt.

Die die Durchflussöffnungen verschließende, unterhalb des Bodens mittels des Umbördelungsmittels fixierte Membrane klappt bei der Flüssigkeitsentnahme, wenn z. B. der Kolben gezogen wird, mit ihrem von dem Umbördelungsmittel nicht erfassten Abschnitt nach unten, in Einströmrichtung zum Röhrcheninneren weg und gibt den Durchfluss frei.

Die Hülsen ermöglichen es, bestehende Verschlusskappen auf einfache Weise mit Rückstromsperren nachzurüsten. Die Hülsen bzw. die Einzelhülsen zusammen mit den Rückstromsperren brauchen dazu lediglich von der offenen Seite der Verschlusskappen her in die Längsbohrung des domartigen Ansatzes eingedrückt zu werden.

Eine weitere Ausführung der Erfindung sieht vor, dass das Haltemittel als ein ringartiger, einen zentrischen Klemmkonus und konzentrisch angeordnet vorgesehene Durchflussöffnungen aufweisender Innenflansch der Längsbohrung des domartigen Ansatzes und die Rückstromsperre als Ventilkörper mit einer ausladenden, elastischen Schirmwand ausgebildet ist, wobei der in den Klemmkonus eingerastete Ventilkörper mit seiner Schirmwand die Durchflussöffnungen überlappt und während der Flüssigkeitsentnahme freigibt.

Das Haltemittel ist hierbei ein von vornherein fester Bestandteil der Verschlusskappe, wobei in die Ringbohrung des Innenflansches dann der die Stirnwand aufweisende Ventilkörper der Rückstromsperre eingedrückt und von dem Klemmkonus fixiert wird.

Die Fixierung des Ventilkörpers in der Ringbohrung des Innenflansches läßt sich nach einer vorteilhaften Ausgestaltung der Erfindung dadurch unterstützen, dass die Schirmwand einen randseitig umlaufenden, nach unten abgewinkelten und radial ausgestellten Außenflansch aufweist. Der schräg nach außen verlaufende Außenflansch presst sich nämlich unter Vorspannung der Schirmwand unterhalb des Innenflansches an die Innenwand der Längsbohrung des domartigen Ansatzes an.

Weitere Einzelheiten und Merkmale der Erfindung ergeben sich aus den Ansprüchen und der nachfolgenden Beschreibung von in den Zeichnungen dargestellten Ausführungseispielen. Es zeigen:
- Fig. 1: als Gesamtansicht ein handelsübliches Vakuumröhrchen mit einer einen domartigen Ansatz aufweisenden Verschlusskappe;
- Fig. 2: in einer explosiven Gesamtansicht ein nach dem Aspirationsprinzip arbeitendes, mit einer Kappe wie in Fig. 1 verschlossenes, handelsübliches (S-Monorette®) Blutentnahmeröhrchen und einen dazu gehörigen, so auch bei dem Röhrchen nach Fig. 1 verwendbaren Kanülenhalter;
- Fig. 3a, 3b: ein Röhrchen wie in Fig. 2 und als vergrößerte Einzelheit (Fig. 3b) eine in dem domartigen Ansatz angeordnete Rückstromsperre;
- Fig. 4a, 4b: ein Röhrchen wie in Fig. 2 und als vergrößerte Einzelheit (Fig. 4b) eine in dem domartigen Ansatz angeordnete Rückstromsperre in einer sogenannten Duckbrill-Ausführung;
- Fig. 5a, 5b: ein Röhrchen wie in Fig. 2 und als vergrößerte Einzelheit (Fig. 5b) eine andere Ausführung einer in dem domartigen Ansatz angeordneten Rückstromsperre;
- Fig. 6a, 6b: ein Röhrchen wie in Fig. 2 und als vergrößerte Einzelheit (Fig. 6b) eine in einem ringartigen, integrierten Innenflansch des domartigen Ansatzes angeordnete Rückstromsperre;
- Fig. 7a, 7b: eine Abwandlung einer wie grundsätzlich in den Figuren 6a, 6b dargestellten Rückstromsperre.

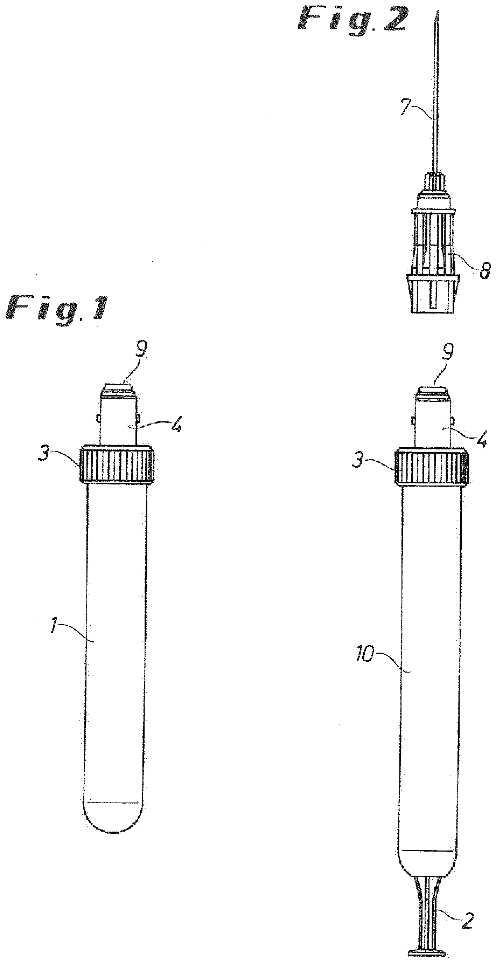

Bei den in den Fig. 1 und 2 dargestellten Röhrchen 1 und 10 handelt es sich um üblich eingesetzte Blutentnahmeröhrchen, wobei das Röhrchen 1 der Fig. 1 nach dem Vakuumprinzip und das Röhrchen 10 der Fig. 2 im Zusammenwirken mit einer aus dem Röhrchenboden herausgeführten Kolbenstange 2 nach dem Aspirationsprinzip arbeitet. In beiden Fällen sind die oben offenen Enden der Röhrchen 1, 10 durch eine Schraub- oder Stopfenkappe 3 verschlossen, die mit einem domartigen Ansatz 4 versehen ist, der mit einer den Durchfluss gewährleistenden Längsbohrung 5 in das Röhrcheninnere mündet und an seinem Außenumfang mehrere Noppen 6 zur Verriegelung mit einem eine Kanüle 7, ggf. Doppelkanüle, tragenden Nadelhalter 8 aufweist (vgl. Fig. 2). Zur Blutentnahme wird der Kanülenhalter 8 auf den domartigen Ansatz 4 aufgeschoben und bajonettartig mit den Noppen 6 verriegelt. Beim Aufschieben bzw. -stecken durchsticht die Kanüle eine in das obere Ende der Längsbohrung 5 eingesetzte Membrane 9.

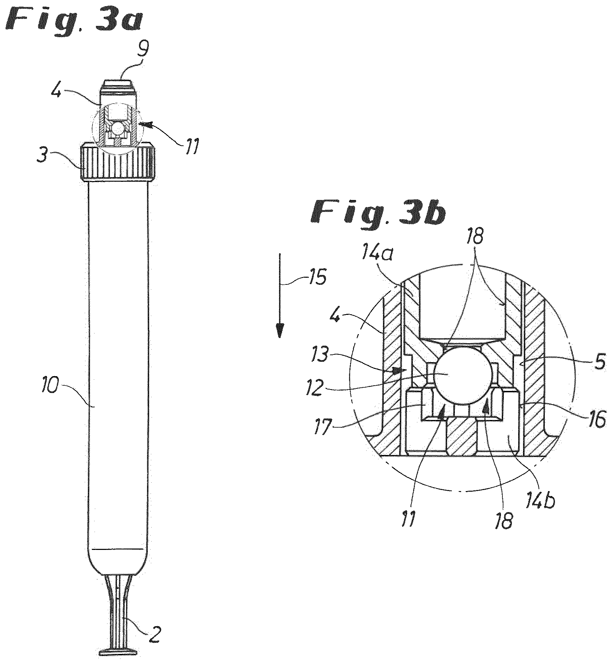

Wie sich aus den verschiedenen Varianten der Fig. 3 bis 7 entnehmen läßt, ist bei allen Ausführungen in der einen Durchmesser von nur wenigen Millimetern besitzenden Längsbohrung 5 des domartigen Ansatzes 4 eine Rückstromsperre 11 ausgebildet.

Nach den Fig. 3a, 3b besteht die Rückstromsperre 11 aus einer von einer (nicht gezeigten) Feder beaufschlagten Kugel 12, die in einem in die Längsbohrung 5 des domartigen Ansatzes 4 eingepassten Haltemittel 13, das sich aus einer oberen Hülse 14a und einer in Einströmrichtung 15 gesehen unteren Hülse 14b zusammensetzt, angeordnet ist. Die untere Hülse 14b ist mit einem kronenkranzartigen Kopfrand 16 ausgebildet, der durch seine Lücken zwischen den Kronenstegen 17 mehrere Durchflussöffnungen 18 bereitstellt. Diese stehen während der Flüssigkeitsentnahme, bei der sich die Kugel 12 aus der Schließposition der Fig. 3b in Einströmrichtung 15 nach unten verlagert hat, in Strömungsverbindung mit der Durchflussöffnung 18 der oberen Hülse 14a.

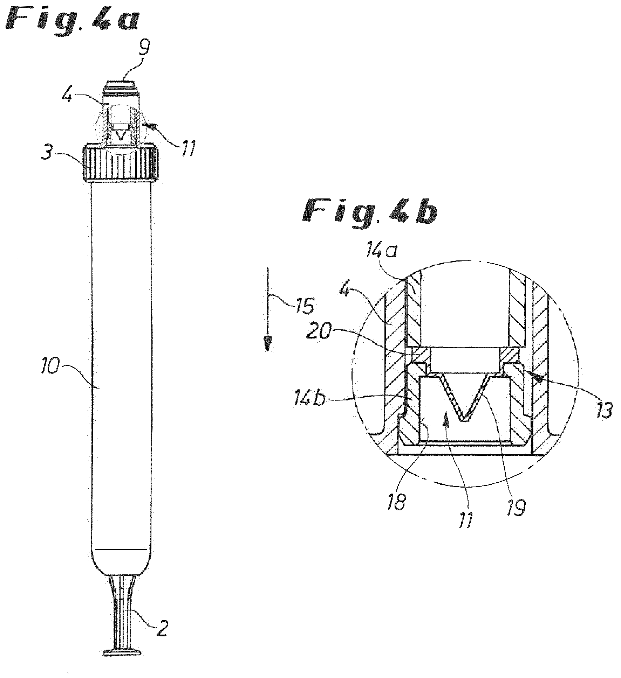

Die Fig. 4a, 4b zeigen ebenfalls die Kombination einer Rückstromsperre mit zwei Hülsen 14a, 14b als Haltemittel 13. Die Rückstromsperre 11 ist als zwischen der oberen und der unteren Hülse 14a, 14b geklemmtes, zentrisch einen in die Durchflussöffnung 18 der unteren Hülse 14b hineinragendes, einen schnabelförmigen Ventilkopf 19 besitzendes Ringelement 20 ausgeführt. Während bzw. bei der Flüssigkeitsentnahme öffnet sich der in Fig. 4b geschlossen dargestellte Schnabel des Ventilkopfes 19 und stellt die Strömungsverbindung zur Durchflussöffnung 18 der oberen Hülse 14a her, so dass die entnommene Flüssigkeit in das Röhrchen 1 bzw. 10 strömen kann.

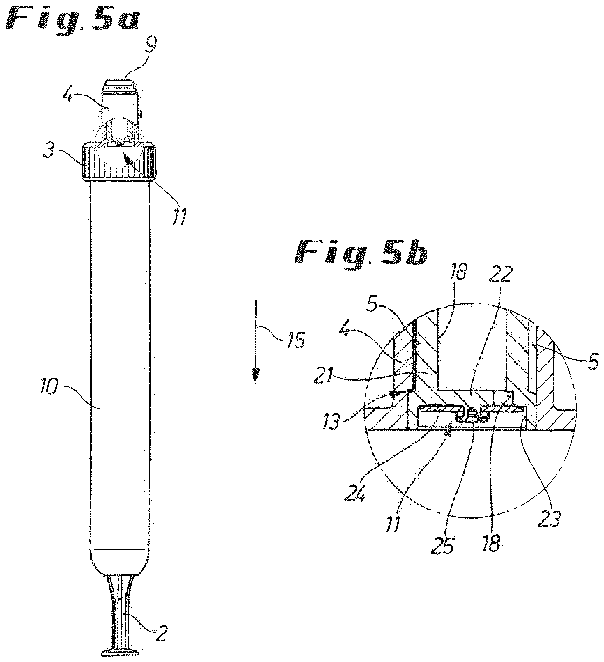

Nach den Fig. 5a, 5b dient eine in die Längsbohrung 5 des domartigen Ansatzes 4 eingepasste Einzelhülse 21 als Haltemittel 13. An dem in Einströmrichtung 15 unteren Ende ist in der im Durchmesser großen Durchflussöffnung 18 der oberen Hülse 14a ein Boden 22 vorgesehen, der einerseits mit mehreren außermittig über den Umfang verteilten, im Durchmesser kleineren Durchflussöffnungen 18 versehen ist und andererseits einen nach unten offenen Ringraum 23 der Einzelhülse 21 begrenzt. Die Rückstromsperre 11 ist hier eine elastische Membranscheibe 24, die die außermittigen Durchflussöffnungen 18 überlappend mittels mittig an der Unterseite des Bodens 22 vorgesehenen Umbördelungsmitteln 25 und somit vom Ringraum 23 her mit Anlage an die Unterseite des Bodens 22 festgelegt bzw. geklemmt wird. Bei der Flüssigkeitsentnahme klappt die Membranscheibe 24 mit ihrem neben dem Umbördelungsmittel 25 liegenden Abschnitt nach unten weg und gibt die Strömungsverbindungen der Durchflussöffnungen 18 frei.

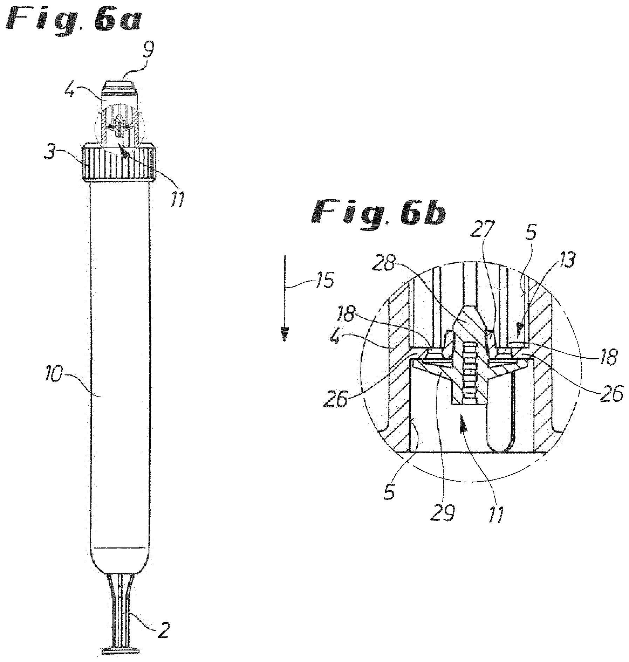

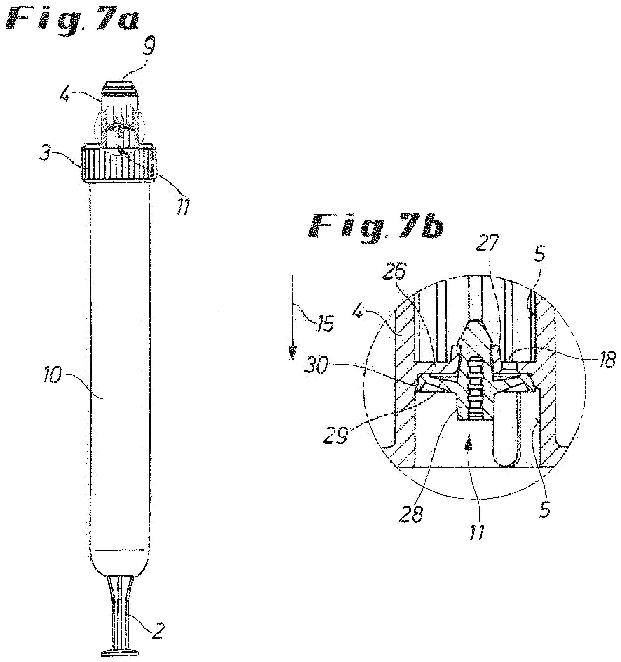

Bei den Entnahmevorrichtungen der Fig. 6, 6b und 7a, 7b ist das Haltemittel 13 als ein in der Längsbohrung 5 des domartigen Ansatzes 4 ausgebildeter, eine Mittenbohrung aufweisender, ringartiger Innenflasch 26 ausgeführt. Gleichwohl könnte das auch eine eingepasste Hülse mit einem Boden und darin vorgesehener Mittenbohrung sein. Die Mittenbohrung des Innenflansches 26 wird von einem Klemmkonus 27 eingefasst und konzentrisch dazu sind über den Umfang des Innenflansches 26 verteilt mehrere Durchflussöffnungen 18 angeordnet. Die Rückstromsperre 11 besteht aus einem in den Klemmkonus 27 befestigten Ventilkörper 28, der eine elastische Schirmwand 29 aufweist, die sich von der Unterseite her die Durchflussöffnungen 18 überlappend an den Innenflansch 26 anlegt. Die Ausgestaltung nach Fig. 7b läßt erkennen, dass die Schirmwand 29 randseitig, an ihrem Außenumfang, einen nach unten abgewinkelten, zur Anlage an die Innenwandung der Längsbohrung 5 des domartigen Ansatzes 4 radial ausgestellten Außenflansch 30 aufweisen kann. Bei bzw. während der Flüssigkeitsentnahme klappt die Schirmwand 29 nach unten in Einströmrichtung 15 weg, wodurch die Durchflussöffnungen 18 des Innenflansches 26 frei gegeben werden und die Strömungsverbindung zwischen der ober- und unterhalb des Innenflansches 26 liegenden Längsbohrung 5 des domartigen Ansatzes 4 und damit zum Röhrcheninneren hergestellt wird.

### Bezugszeichenliste

- 1: Röhrchen / Blutentnahmeröhrchen (Vakuumprinzip)
- 2: Kolbenstange
- 3: Schraub- oder Stopfenkappe / Kappe
- 4: domartiger Ansatz
- 5: Längsbohrung (des Ansatzes 4)
- 6: Noppen (Verriegelungsnoppen)
- 7: Kanüle (Doppelkanüle)
- 8: Nadelhalter
- 9: Membrane
- 10: Röhrchen / Blutentnahmeröhrchen (Aspirationsprinzip)
- 11: Rückstromsperre
- 12: Kugel
- 13: Haltemittel
- 14a: obere Hülse
- 14b: untere Hülse
- 15: Einströmrichtung (Pfeil)
- 16: kronenkranzartiger Kopfrand
- 17: Kronensteg
- 18: Durchflussöffnung
- 19: Ventilkopf
- 20: Ringelement
- 21: Einzelhülse
- 22: Boden
- 23: Ringraum
- 24: Membranscheibe
- 25: Umbördelungsmittel
- 26: Innenflansch
- 27: Klemmkonus
- 28: Ventilkörper
- 29: Schirmwand
- 30: Außenflansch

## Patentansprüche

1. Vorrichtung zur Ent- und / oder Aufnahme von Körperflüssigkeiten, insbesondere Blut, umfassend ein Röhrchen (1), das an seinem oberen, offenen Ende mit einer Kappe (3) dicht verschlossen ist, die mit einer Rückstromsperre (11) ausgebildet ist und zum Halten einer von einem Nadelhalter (8) getragenen Kanüle bzw. Doppelkanüle (7) einen domartigen Ansatz (4) aufweist, der an seinem oberen Ende in einer Längsbohrung (5) eine von der Kanüle (7) durchstechbare Membrane (9) aufnimmt,
**dadurch gekennzeichnet,**
**dass** die Rückstromsperre (11) in dem domartigen Ansatz (4) mit einem in der Längsbohrung (5) unterhalb der durchstechbaren Membrane (9) vorgesehenen Haltemittel (13) angeordnet ist, wobei das Haltemittel (13) mit mindestens einer durch die Rückstromsperre (11) nach der Flüssigkeitsentnahme zum Röhrcheninneren verschlossenen Durchflussöffnung (18) ausgebildet ist.

2. Vorrichtung nach Anspruch 1,
**dadurch gekennzeichnet,**
**dass** das Haltemittel (13) aus zwei axial aufeinanderfolgenden, durch Klemmung in der der Längsbohrung (5) angeordneten Hülsen (14a; 14b) besteht, wobei die Rückstromsperre (11) im Übergang von der in Einströmrichtung (15) oberen Hülse (14a) und der dieser folgenden unteren Hülse (14b) vorgesehen ist.

3. Vorrichtung nach Anspruch 2,
**dadurch gekennzeichnet,**
**dass** die untere Hülse (14b) mit einem kronenartigen Kopfrand (16)) und die Rückstromsperre (11) als federbeaufschlagte Kugel (12) ausgebildet ist, wobei sich die Kugel (12) während der Flüssigkeitsentnahme aus ihrer die Durchflussöffnung (18) der oberen Hülse (14a) verschließenden Position nach unten in die den Durchfluss freigebende Position zwischen den Kronenstegen (17) des Kopfrandes (16) der unteren Hülse (14b) verlagert.

4. Vorrichtung nach Anspruch 2,
**dadurch gekennzeichnet,**
**dass** zwischen der oberen und der unteren Hülse (14a, 14b) als Rückstromsperre (11) ein einen schnabelförmigen, in die Durchflussöffnung (18) der unteren Hülse (14b) hineinragenden, sich bei der Flüssigkeitsentnahme weitenden und danach wieder schließenden, Ventilkopf (19) aufweisendes Ringelement (20) vorgesehen ist.

5. Vorrichtung nach Anspruch 1,
**dadurch gekennzeichnet,**
**dass** das Halteelement (13) eine Einzelhülse (21) ist, die endseitig, unterhalb eines mit außermittigen Durchflussöffnungen (18) versehenen Bodens (22) einen Ringraum (23) aufweist, der als Rückstromsperre (11) eine von mittig am Boden (22) angeordneten Umbördelungsmitteln (25) geklemmte, in dem Ringraum (23) die außermittigen Durchflussöffnungen (18) überlappende, elastische Membranscheibe (24) aufnimmt, wobei die Membranscheibe (24) während der Flüssigkeitsentnahme nach unten wegklappt und die Durchflussöffnungen (18) freigibt.

6. Vorrichtung nach Anspruch 1,
**dadurch gekennzeichnet,**
**dass** das Haltemittel (13) als ein ringartiger, einen zentrischen Klemmkonus (27) und konzentrisch dazu angeordnete Durchflussöffnungen (18) aufweisender Innenflansch (26) der Längsbohrung (5) des domartigen Ansatzes (5) und die Rückstromsperre (11) als Ventilkörper (28) mit einer ausladenden, elastischen Schirmwand (29) ausgebildet ist, wobei der in den Klemmkonus (27) eingerastete Ventilkörper (28) mit seiner Schirmwand (29) die Durchflussöffnungen (18) überlappt und während der Flüssigkeitsentnahme freigibt.

7. Vorrichtung nach Anspruch 6,
**dadurch gekennzeichnet,**
**dass** die Schirmwand (29) einen randseitig umlaufenden, nach unten abgewinkelten und radial ausgestellten Außenflansch (30) aufweist.

## Claims

1. A device for removing and/or collecting bodily fluids, in particular blood, comprising a tube (1) which is tightly closed at the upper open end thereof with a cap (3) which is configured to have a non-return valve (11) and which has a dome-like attachment (4) for retaining a cannula or double canula (7) carried by a needle holder (8), the upper end of the attachment accommodating a membrane (9) which can be pierced by the cannula (7), in a longitudinal bore (5), **characterized in that**
the non-return valve (11) is disposed in the dome-like attachment (4) by means of a retaining means (13) which is provided in the longitudinal bore (5) below the pierceable membrane (9), wherein the retaining means (13) is configured with at least one outlet (18) which is closed to the interior of the tube by the non-return valve (11) after removing the fluid.

2. The device as claimed in claim 1,
**characterized in that**
the retaining means (13) consists of two axially consecutive bushings (14a; 14b) disposed in the longitudinal bore (5) by clamping, wherein the non-return valve (11) is provided in the transition between the upper bushing (14a) in the direction of flow (15) and the lower bushing (14b) which follows it.

3. The device as claimed in claim 2,
**characterized in that**
the lower bushing (14b) is configured with a crown-like top edge (16) and the non-return valve (11) is configured as a spring-loaded ball (12), wherein during fluid removal, the ball (12) is displaced from its position closing the outlet (18) of the upper bushing (14a) downwardly into the position opening a passage for flow between the uprights of the crown (17) of the top edge (16) of the lower bushing (14b).

4. The device as claimed in claim 2,
**characterized in that**
between the upper and the lower bushing (14a, 14b), as the non-return valve (11), a ring element (20) is provided which has a valve head (19) which is in the shape of a beak, which protrudes into the outlet (18) of the lower bushing (14b), which opens during fluid removal and which closes again afterwards.

5. The device as claimed in claim 1,
**characterized in that**
the retaining element (13) is a single bushing (21) the end of which has a circular chamber (23) below a base (22) provided with eccentric outlets (18) which, as the non-return valve (11), accommodates a resilient membrane disk (24) clamped with bead means (25) disposed centrally on the base (22) and which overlaps the eccentric outlets (18) in the circular chamber (23), wherein the membrane disk (24) tilts downwards during fluid removal and exposes the outlets (18).

6. The device as claimed in claim 1,
**characterized in that**
the retaining means (13) is configured as a ring-like internal flange (26) of the longitudinal bore (5) of the dome-like attachment (5) having a central clamping cone (27) and outlets (18) disposed concentrically thereto and the non-return valve (11) is configured as a valve body (28) with a protruding resilient shielding wall (29), wherein the shielding wall (29) of the valve body (28) engaged in the clamping cone (27) overlaps the outlets (18) which are exposed during fluid removal.

7. The device as claimed in claim 6,
**characterized in that**
the shielding wall (29) has an outer flange (30) which passes around the edge and is inclined downwardly and radially thereto.

## Revendications

1. Dispositif destiné à prélever et/ou à recueillir des liquides corporels, en particulier du sang, comprenant un petit tube (1), qui est hermétiquement fermé à son extrémité supérieure, ouverte par un bouchon (3), qui est constitué avec un clapet antiretour (11) et comporte un embout (4) en forme de dôme pour maintenir une canule ou canule double (7) supportée par un porte-aiguille (8), qui reçoit à son extrémité supérieure dans un trou longitudinal (5) une membrane (9) pouvant être perforée par la canule (7),
**caractérisé en ce que**
le clapet antiretour (11) est disposé dans l'embout en forme de dôme (4) avec un moyen de maintien (13) prévu dans le trou longitudinal (5) en dessous de la membrane (9) pouvant être percée, sachant que le moyen de maintien (13) est constitué avec au moins une ouverture de passage (18) fermée vers l'intérieur du petit tube par le clapet antiretour (11) après le prélèvement de liquide.

2. Dispositif selon la revendication 1,
**caractérisé en ce que**
le moyen de maintien (13) est composé de deux manchons (14a, 14b) se succédant axialement, disposés par serrage dans le trou longitudinal (5), sachant que le clapet antiretour (11) est prévu au passage de transition du manchon supérieur (14a) en direction de l'entrée (15) et le manchon inférieur (14b) suivant celui-ci.

3. Dispositif selon la revendication 2,
**caractérisé en ce que**
le manchon inférieur (14b) est constitué avec un bord de tête de type couronne (16) et le clapet antiretour (11) comme une sphère sous effet de ressort (12), sachant que la sphère (12) se déplace vers le bas pendant le prélèvement de liquide de sa position fermant l'ouverture de passage (18) du manchon supérieur (14a) dans la position libérant le passage entre les nervures de couronne (17) du bord de tête (16) du manchon inférieur (14b).

4. Dispositif selon la revendication 2,
**caractérisé en ce qu'**
entre le manchon supérieur et le manchon inférieur (14a, 14b) un élément annulaire (20) est prévu en tant que clapet antiretour (11) comportant un chapeau de soupape (19) en forme de bec, pénétrant dans l'ouverture de passage (18) du manchon inférieur (14b) s'élargissant lors du prélèvement de liquide et se fermant à nouveau ensuite.

5. Dispositif selon la revendication 1,
**caractérisé en ce que**
l'élément de maintien (13) est un manchon individuel (21), qui comporte un espace annulaire (23) en extrémité, en dessous d'un fond (22) doté d'ouvertures de passage (18) excentrées, qui reçoit en tant que clapet antiretour (11) un disque de membrane (24) élastique, serré par des moyens de bord rabattu (25) disposés au centre sur le fond (22), recouvrant dans l'espace annulaire (23) les ouvertures de passage (18) excentrées, sachant que le disque de membrane (24) se rabat vers le bas pendant le prélèvement de liquide et libère les ouvertures de passage (18).

6. Dispositif selon la revendication 1,
**caractérisé en ce que**
le moyen de maintien (13) est constitué comme une bride interne (26) du trou longitudinal (5) de l'embout en forme de dôme (5), comportant un cône de serrage central de type annulaire (27) et des ouvertures de passage (18) disposées de façon concentrique à cette effet et le clapet antiretour (11) est constitué comme corps de soupape (28) avec une paroi d'isolation (29) élastique en saillie, sachant que le corps de soupape (28) enclenché dans le cône de serrage (27) recouvre avec sa paroi d'isolation (29) les ouvertures de passage (18) et les libère pendant le prélèvement de liquide.

7. Dispositif selon la revendication 6,
**caractérisé en ce que**
la paroi d'isolation (29) comporte une bride extérieure (30) périphérique en bordure recourbée vers le bas et radialement exposée.
